(19) Europäisches Patentamt
European Patent Office
Office européen des brevets

(11) Veröffentlichungsnummer: **0 550 788 A1**

# (12) EUROPÄISCHE PATENTANMELDUNG

(21) Anmeldenummer: **92100313.3**

(51) Int. Cl.[5]: **C07C 271/22**, A01N 47/12

(22) Anmeldetag: **10.01.92**

(43) Veröffentlichungstag der Anmeldung:
**14.07.93 Patentblatt 93/28**

(84) Benannte Vertragsstaaten:
**BE CH DE ES FR GB GR IT LI NL**

(71) Anmelder: **BAYER AG**

**W-5090 Leverkusen 1 Bayerwerk(DE)**

(72) Erfinder: **Seitz, Thomas, Dr.**
**Mozartstrasse 32**
**W-4019 Monheim(DE)**
Erfinder: **Wollweber, Detlef, Dr.**
**Paul-Ehrlich-Strasse 17**
**W-5600 Wuppertal 1(DE)**
Erfinder: **Brandes, Wilhelm, Dr.**
**Eichendorffstrasse 3**
**W-5653 Leichlingen 1(DE)**
Erfinder: **Dehne, Heinz-Wilhelm, Dr.**
**Krischer Strasse 81**
**W-4019 Monheim(DE)**

## (54) Substituierte Valinamid-Derivate.

(57) Beschrieben werden neue Valinamid-Derivate der Formel (I)

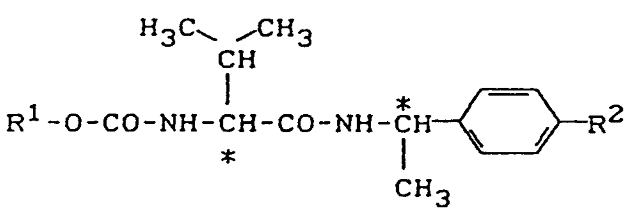

(I)

in welcher
$R^1$ für i-Propyl oder s-Butyl und
$R^2$ für Chlor, Methyl, Ethyl oder Methoxy steht,
deren Herstellung sowie deren Verwendung als Schädlingsbekämpfungsmittel.

EP 0 550 788 A1

Die Erfindung betrifft neue Valinamid-Derivate, ein Verfahren zu ihrer Herstellung sowie ihre Verwendung als Schädlingsbekämpfungsmittel, insbesondere als Fungizide.

Die erfindungsgemäßen Substanzen besitzen eine ausgezeichnete Wirkung bei der Bekämpfung von Schädlingen. Insbesondere können sie als Fungizde, vor allem im Pflanzenschutz verwendet werden.

Bestimmte Aminosäureamide sind bereits bekannt (vgl. z.Bsp. EP-A 236 874). Eine Verwendung dieser Verbindungen in Schädlingsbekämpfungsmitteln wird jedoch nicht beschrieben.

Es wurden nun neue Valinamid-Derivate der allgemeinen Formel (I)

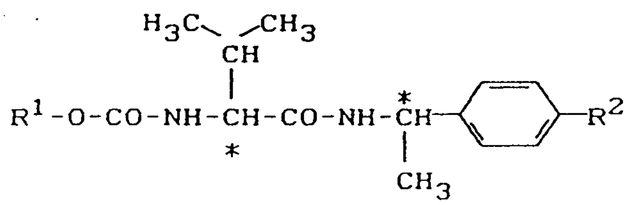

(I)

in welcher

$R^1$ für i-Propyl oder s-Butyl und

$R^2$ für Chlor, Methyl, Ethyl oder Methoxy steht

gefunden.

Die Verbindungen der Formel (I) enthalten zwei Chiralitätszentren und können somit in verschiedenen Enantiomeren- und Diastereomerengemischen vorliegen, die gegebenenfalls in üblicher Art und Weise getrennt werden können. Sowohl die reinen Enantiomeren und Diasteromeren, als auch die Gemische werden erfindungsgemäß beansprucht.

Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Man erhält die Valinamid-Derivate der allgemeinen Formel (I)

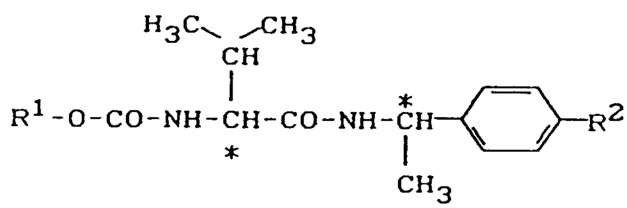

(I)

in welcher

$R^1$ für i-Propyl oder s-Butyl und

$R^2$ für Chlor, Methyl, Ethyl oder Methoxy steht

wenn man

eine substituierte Aminosäure der Formel (II)

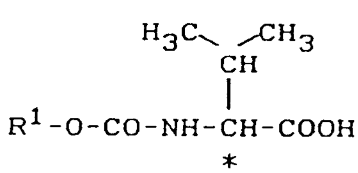

(II)

in welcher

$R^1$ für i-Propyl oder s-Butyl steht, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels mit einem Amin der Formel (III)

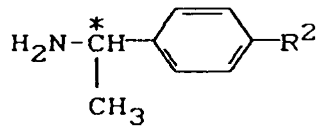

(III)

in welcher

$R^2$ für Chlor, Methyl, Ethyl oder Methoxy steht,

umsetzt.

Verwendet man beispielsweise i-Propyloxycarbonyl-L-valin und 4-Chlorphenethylamin als Ausgangsprodukte,
so kann der Ablauf des erfindungsgemäßen Verfahrens durch das folgende Formelschema wiedergegeben werden:

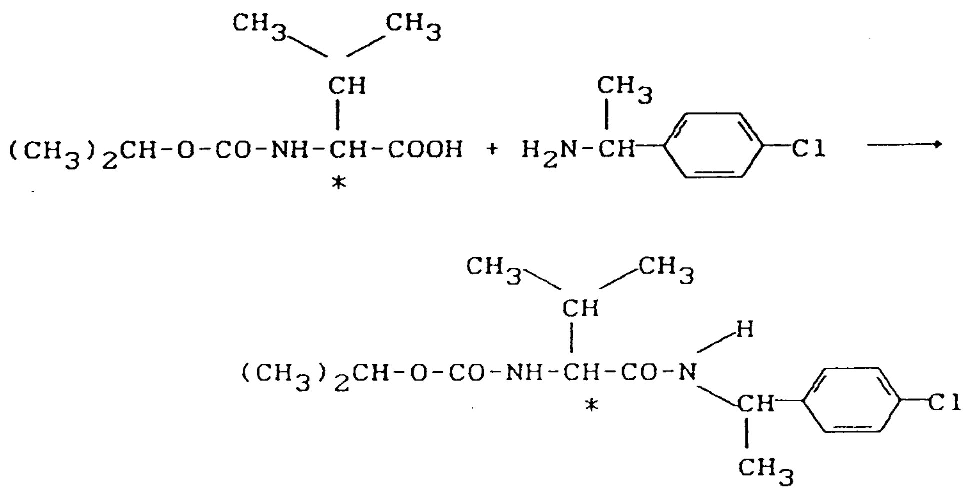

Bevorzugt sind diejenigen Verbindungen der Formel (I), in welchen die zugrundeliegende Aminosäure der Formel (II) i-Propyloxycarbonyl-L-valin oder s-Butoxycarbonyl-L-valin ist und
das eingesetzte Phenethylamin der Formel (III),
in welchem

$R^2$ für Chlor, Methyl, Ethyl oder Methoxy steht,

entweder racemisch ist oder
die R(+) Konfiguration oder
die S(-) Konfiguration
am asymmetrischen Zentrum aufweist.

Besonders bevorzugt sind diejenigen Verbindungen der Formel (I)
in welchen
die zugrundeliegende Aminosäure i-Propyloxy-carbonyl-L-valin oder s-Butoxycarbonyl-L-valin ist und
das eingesetzte Phenethylamin
in welchem

$R^2$ für Chlor, Methyl, Ethyl oder Methoxy steht

entweder racemisch ist oder am asymmetrischen Zentrum die R(+)-Konfiguration aufweist.

Die für die Durchführung des erfindungsgemäßen Verfahrens als Ausgangsstoffe zu verwendenden Aminosäure-Derivate sind durch die Formel (II) allgemein definiert. In dieser Formel haben $R^1$ und $R^2$ vorzugsweise die Bedeutungen, die bereits im Zusammenhang mit der Beschreibung der erfindungsmäßen Stoffe der Formel (I) für diese Substituenten bevorzugt genannt wurden.

Die Aminosäure-Derivate der Formel (II) sind allgemein bekannt (vgl. z.B. Houben-Weyl, Methoden der organischen Chemie, Band XV, Teil 1 und 2, Seiten 46 ff und 112 ff, Georg Thieme Verlag, Stuttgart 1974; D. Keller et.al., Org. Synth. 60, 2145 (1981); bzw. R.C. Sheppard, A Specialist Periodical Report, Amino-acids, Peptids and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E.

Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens als Ausgangsstoffe zu verwendenden carboxy-aktivierten Derivate der Aminosäure der Formel (II) sind allgemein bekannt.

Als carboxy-aktivierte Derivate der Aminosäuren der Formel (II) kommen alle carboxy-aktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Die Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid, Nitrile, wie z.B. Acetonitril, Chlorkohlenwasserstoffe, wie z.B. Methylenchlorid, Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.

Die außerdem für die Durchführung des erfindungsgemäßen Verfahrens als Ausgangsstoffe zu verwendenden Amine sind durch die Formel (III) allgemein definiert In diesen Formeln hat $R^2$ die oben genannten Bedeutungen.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylpiperidin, sowie anorganische Basen, z.B. Metallhydroxide wie Natrium- und Kaliumhydroxid oder Metallcarbonate wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens in einem größeren Bereich variiert werden. Im allgemeinen arbeitet man zwischen -78 bis +120 °C, vorzugsweise bei -60 bis +40 °C.

Bei der Durchführung des erfindungsgemäßen Verfahrens arbeitet man vorzugsweise in äquimolaren Mengen.

Dabei werden die Aminosäurederivate der Formel (II) als reine optische Isomere (D- bzw. L-Form) oder als Racemate eingesetzt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Camphersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;

Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Phytophthora-Arten an Tomaten oder Plasmopara-Arten an Reben einsetzen.

Die Wirkstoffe zeigen darüberhinaus auch eine blattinsektizide Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und

Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Bei der Behandlung von tierischen Schädlingen liegen im allgemeinen die Wirkstoffkonzentrationen zwischen 0,0000001 bis zu 95 % Wirkstoff, vorzugsweise zwischen 0,0001 bis 1 %.

## Herstellungsbeispiele

### Beispiel 1

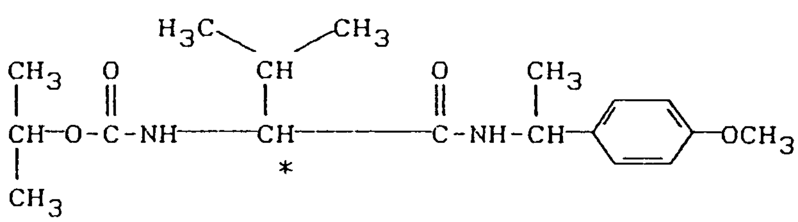

Zu 4,67 g i-Propoxycarbonyl-L-valin (0,023 Mol), gelöst in 50 ml $CH_2Cl_2$, wurden bei -20 °C 2,3 g (0,023 Mol) N-Methylpiperidin zugegeben. Anschließend tropft man bei -20 °C schnell 3,2 g (0,023 Mol) Chlorameisensäureisobutylester zu, rührt bei gleicher Temperatur 10 Minuten nach, kühlt auf -60 °C ab und läßt 3,5 g (0,023 Mol) 4-Methoxy-1-phenylethylamin zulaufen, wobei die Temperatur unter -15 °C gehalten wird. Nach 2 Stunden bei -15 °C läßt man 15 Stunden bei Raumtemperatur nachrühren, filtriert vom Feststoff ab, wäscht mit $CH_2Cl_2$ nach, engt ein, gibt den Rückstand auf Wasser, extrahiert 2mal mit Essigester, wäscht die vereinigten Essigester-Phasen mit $NaHCO_3$-Lösung und Wasser, trocknet und engt ein. Man erhält 4,64 g (60 % d. Th.) N-(i-Propyloxycarbonyl)-L-valin-4-methoxyphenylethylamid mit einem Schmelzpunkt von 167 °C.

Analog Beispiel 1 werden die folgenden Verbindungen der Formel (I) erhalten

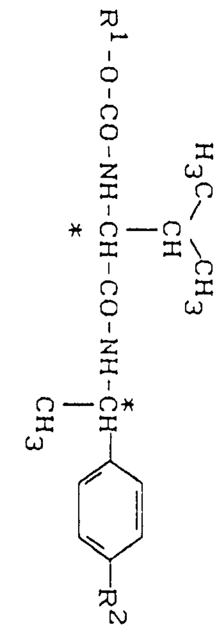

(I)

Tabelle 1:

| Bsp. Nr. | $R^1$ | $R^2$ | physikalische Konstante | eingesetzte Aminosäure |
|---|---|---|---|---|
| 2 | $-CH(CH_3)_2$ | $-Cl$ | Fp: 176° C | i-Propyloxycarbonyl-L-valin |
| 3 | $-CH(CH_3)_2$ | $-CH_3$ | Fp: 160° C | i-Propyloxycarbonyl-L-valin |
| 4 | $-CH(CH_3)_2$ | $-Cl$ | Fp: 170° C | i-Propyloxycarbonyl-L-valin (R+) Amid |
| 5 | $-CH\genfrac{}{}{0pt}{}{CH_3}{C_2H_5}$ | $-Cl$ | Fp: 166° C | s-Butyloxycarbonyl-L-valin |
| 6 | $-CH\genfrac{}{}{0pt}{}{CH_3}{C_2H_5}$ | $-CH_3$ | Fp: 143° C | s-Butyloxycarbonyl-L-valin |
| 7 | $-CH\genfrac{}{}{0pt}{}{C_2H_5}{CH_3}$ | $-OCH_3$ | Fp: 153° C | s-Butyloxycarbonyl-L-valin |

Tabelle 1: - Fortsetzung

| Bsp. Nr. | $R^1$ | $R^2$ | physikalische Konstante | eingesetzte Aminosäure |
|---|---|---|---|---|
| 8 | 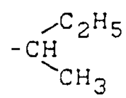 | $-Cl$ | Fp:168° C | s-Butyloxycarbonyl-L-valin (R+) Amid |
| 9 | $-CH(CH_3)_2$ | $-C_2H_5$ | Fp:121-122° C | i-Propyloxycarbonyl-L-valin |
| 10 | 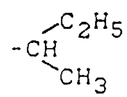 | $-C_2H_5$ | Fp:111-112° C | s-Butyloxycarbonyl-L-valin |
| 11 | $-CH(CH_3)_2$ | $-C_2H_5$ | Fp:154-156° C | i-Propyloxycarbonyl-L-valin (R+) Amid |
| 12 | 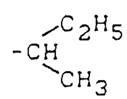 | $-C_2H_5$ | Fp:140-142° C | s-Butyloxycarbonyl-L-valin (R+) Amid |
| 13 | $-CH(CH_3)_2$ | $-CH_3$ | Fp:177-179° C | i-Propyloxycarbonyl-L-valin (R+) Amid |

Tabelle 1: - Fortsetzung

| Bsp. Nr. | $R^1$ | $R^2$ | physikalische Konstante | eingesetzte Aminosäure |
|---|---|---|---|---|
| 14 | 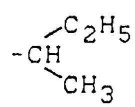 | —$CH_3$ | Fp:169-171° C | s-Butyloxycarbonyl-L-valin (R+) Amid |
| 15 | -$CH(CH_3)_2$ | —$OCH_3$ | Fp:183-185° C | i-Propyloxycarbonyl-L-valin (R+) Amid |
| 16 | 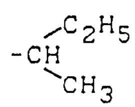 | —$OCH_3$ | Fp:178-180° C | s-Butyloxycarbonyl-L-valin (R+) Amid |

Tabelle 1: - Fortsetzung

| Bsp. Nr. | $R^1$ | $R^2$ | physikalische Konstante | eingesetzte Aminosäure |
|---|---|---|---|---|
| 17 | $-CH(CH_3)_2$ | $-Cl$ | 162° C | i-Propyloxycarbonyl-D,L-valin |
| 18 | $-CH(CH_3)_2$ | $-Cl$ | 168° C | i-Propyloxycarbonyl-D,L-valin ($R^+$) Amid |
| 19 | $-CH(CH_3)_2$ | $-CH_3$ | 140° C | i-Propyloxycarbonyl-D,L-valin |
| 20 | $-CH(CH_3)_2$ | $-OCH_3$ | 138° C | i-Propyloxycarbonyl-D,L-valin |
| 21 | $-CH(CH_3)_2$ | $-CH_2CH_3$ | 130° C | i-Propyloxycarbonyl-D,L-valin |
| 22 | $-CH(CH_3)_2$ | $-CH_3$ | 147° C | i-Propyloxycarbonyl-D,L-valin ($R^+$) Amid |

Anwendungsbeispiele:

Beispiel A

Phytophthora-Test (Tomate)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

In diesem Test zeigen z.B. die Verbindungen gemäß den Herstellungsbeispielen (1), (2), (3), (4), (5), (6), (7), (8), (9), (10), (11), (12), (13), (14), (15) und (16) eine ausgezeichnete fungizide Wirksamkeit.

Beispiel B

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

In diesem Test zeigen z.B. die Verbindungen gemäß den Herstellungsbeispielen (1), (2), (3), (4), (5), (6), (7), (8), (9), (10), (11), (12), (13), (14), (15) und (16) eine ausgezeichnete fungizide Wirksamkeit.

## Patentansprüche

**1.** Valinamid-Derivate der Formel (I)

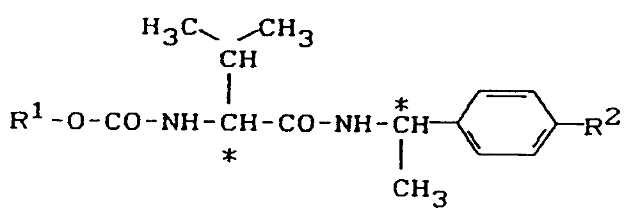

(I)

in welcher
$R^1$ für i-Propyl oder s-Butyl und
$R^2$ für Chlor, Methyl, Ethyl oder Methoxy steht.

**2.** Valinamid-Derivate gemäß Anspruch 1, wobei die zugrundeliegende Aminosäure i-Propyloxycarbonyl-L-valin oder s-Butoxycarbonyl-L-valin ist und

das eingesetzte Phenethylamin, in welchem
$R^2$ für Chlor, Methyl, Ethyl oder Methoxy steht,
entweder racemisch ist oder
die R(+) Konfiguration oder
die S(-) Konfiguration

am asymmetrischen Zentrum aufweist.

**3.** Valinamid-Derivate gemäß Anspruch 1, wobei

die zugrundeliegende Aminosäure i-Propyloxycarbonyl-L-valin oder s-Butoxycarbonyl-L-valin ist und

das eingesetzte Phenethylamin
in welchem
$R^2$ für Chlor, Methyl, Ethyl oder Methoxy steht
entweder racemisch ist oder am asymmetrischen Zentrum die R(+)-Konfiguration aufweist.

**4.** Verfahren zur Herstellung von Valinamid-Derivaten der allgemeinen Formel

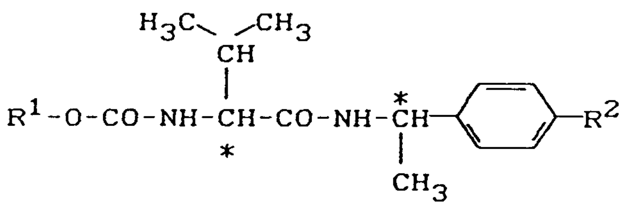

(I)

in welcher
$R^1$ für i-Propyl oder s-Butyl und
$R^2$ für Chlor, Methyl, Ethyl oder Methoxy steht
dadurch gekennzeichnet, daß man

eine substituierte Aminosäure der Formel (II)

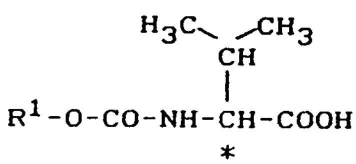

(II)

in welcher
$R^1$ für i-Propyl oder s-Butyl steht, bzw. deren carboxy-aktivierte Derivate,
gegebenenfalls in Gegenwart eines Katalysators, gegebenenfalls in Gegenwart eines Säurebindemittels und gegebenenfalls in Gegenwart eines Verdünnungsmittels mit einem Amin der Formel (III)

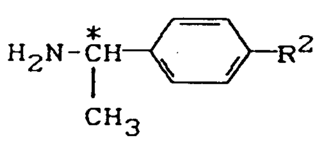

(III)

in welcher
$R^2$ für Chlor, Methyl, Ethyl oder Methoxy steht,
umsetzt.

**5.** Schädlingsbekämpfungsmittel, gekennzeichnet durch einen Gehalt an mindestens einem Valinamid-Derivat der Formel (I) nach den Ansprüchen 1 bis 4.

**6.** Verwendung von Valinamid-Derivaten der Formel (I) nach den Ansprüchen 1 bis 4 zur Bekämpfung von Schädlingen.

**7.** Verfahren zur Bekämpfung von Schädlingen, dadurch gekennzeichnet, daß man Valinamid-Derivate der Formel (I) nach den Ansprüchen 1 bis 4 auf Schädlinge und/oder ihren Lebensraum einwirken läßt.

**8.** Verfahren zur Herstellung von Schädlingsbekämpfungsmitteln, dadurch gekennzeichnet, daß man Valinamid-Derivate der Formel (I) nach den Ansprüchen 1 bis 4 mit Streckmitteln und/oder oberflächenaktiven Mitteln vermischt.

Europäisches
Patentamt

# EUROPÄISCHER RECHERCHENBERICHT

Nummer der Anmeldung

EP 92 10 0313

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-0 398 072 (BAYER AG)<br>* Seite 4, Zeile 36 - Zeile 37; Ansprüche *<br>--- | 1,4-8 | C07C271/22<br>A01N47/12 |
| E | EP-A-0 472 996 (BAYER AG)<br>* Ansprüche *<br>----- | | |

| RECHERCHIERTE SACHGEBIETE (Int. Cl.5 ) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 31 AUGUST 1992 | SANCHEZ GARCIA J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)